Europäisches Patentamt

European Patent Office

Office européen des brevets

(19)

(11) **EP 1 282 438 B1**

(12) **EUROPEAN PATENT SPECIFICATION**

(45) Date of publication and mention
of the grant of the patent:
**03.08.2005 Bulletin 2005/31**

(21) Application number: **01931458.2**

(22) Date of filing: **10.05.2001**

(51) Int Cl.7: **A61K 38/36**, A61P 7/04

(86) International application number:
**PCT/DK2001/000322**

(87) International publication number:
**WO 2001/085198 (15.11.2001 Gazette 2001/46)**

(54) **USE OF PHARMACEUTICAL COMPOSITION COMPRISING A FACTOR VIIA AND A FACTOR XIII**

VERWENDUNG VON FAKTOR VII-A UND FAKTOR XIII ENTHALTENDEN PHARMAZEUTISCHEN
VERBINDUNGEN

UTILIASATION D'UNE COMPOSITION PHARMACEUTIQUE COMPRENANT UN FACTEUR VIIA
ET UN FACTEUR XIII

(84) Designated Contracting States:
**AT BE CH CY DE DK ES FI FR GB GR IE IT LI LU
MC NL PT SE TR**

(30) Priority: **10.05.2000  DK 200000778
10.05.2000  DK 200000771
06.06.2000  DK 200000871**

(43) Date of publication of application:
**12.02.2003  Bulletin 2003/07**

(60) Divisional application:
**05007833.6**

(73) Proprietor: **Novo Nordisk Health Care AG
8050 Zürich (CH)**

(72) Inventors:
• **PETERSEN, Lars, Christian
DK-2970 Horsholm (DK)**
• **HEDNER, Ulla
S-216 18 Malmo (SE)**
• **ROJKJAER, Rasmus
DK-2820 Gentofte (DK)**

(74) Representative: **Stahr, Pia et al
Novo Nordisk A/S,
Corporate Patents,
Novo Allé
2880 Bagsvaerd (DK)**

(56) References cited:
**EP-A2- 0 225 160          WO-A1-93/06855
WO-A1-93/12813          WO-A1-96/28153
WO-A1-97/29792          WO-A1-99/32143
WO-A2-99/58699**

**Description**

FIELD OF THIS INVENTION

The present invention relates to the use of a combination of a factor VIIa with a factor XIII for the manufacture of a medicament for treatment of subjects suffering from bleeding episodes, or prevention hereof.

BACKGROUND OF THE INVENTION

Haemostasis is initiated by the formation of a complex between tissue factor (TF) being exposed to the circulating blood following an injury to the vessel wall, and FVIIa which is present in the circulation in an amount corresponding to about 1% of the total FVII protein mass. This complex is anchored to the TF-bearing cell and activates FX into FXa and FIX into FIXa on the cell surface. FXa activates prothrombin to thrombin, which activates FVIII, FV, FXI and FXIII. Furthermore, the limited amount of thrombin formed in this initial step of haemostasis also activates the platelets. Following the action of thrombin on the platelets these change shape and expose charged phospholipids on their surface. This activated platelet surface forms the template for the further FX activation and the full thrombin generation. The further FX activation on the activated platelet surface occurs via a FIXa-FVIIIa complex formed on the surface of the activated platelet, and FXa then converts prothrombin into thrombin while still on the surface. Thrombin then converts fibrinogen into fibrin which is insoluble and which stabilizes the initial platelet plug. This process is compartmentalized, i.e., localised to the site of TF expression or exposure, thereby minimizing the risk of a systemic activation of the coagulation system. The insoluble fibrin forming the plug is furthermore stabilised by FXIII-catalysed cross-linking of the fibrin fibres.

FVIIa exists in plasma mainly as a single-chain zymogen, which is cleaved by FXa into its two-chain, activated form, FVIIa. Recombinant activated factor VIIa (rFVIIa) has been developed as a pro-haemostatic agent. The administration of rFVIIa offers a rapid and highly effective pro-haemostatic response in haemophilic subjects with bleedings who cannot be treated with coagulation factor products due to antibody formation. Also bleeding subjects with a factor VII deficiency or subjects having a normal coagulation system but experiencing excessive bleeding can be treated successfully with FVIIa. In these studies, no unfavourable side effects of rFVIIa (in particular the occurrence of thromboembolism) has been encountered.

Extra exogenously administered FVIIa increases the formation of thrombin on the activated platelet surface. This occurs in haemophiliac subjects lacking FIX or FVIII and therefore missing the most potent pathway for full thrombin formation. Also in the presence of a lowered number of platelets or platelets with a defect function, extra FVIIa increases the thrombin formation.

FXIII, the fibrin stabilising factor, is a transglutaminase that cross-links the fibrin monomers thereby providing a fibrin structure with increased resistance to the dissolution by plasmin and other proteolytic enzymes, factor XIII is also known as "fibrinoligase" and "fibrin stabilizing factor". When activated, FXIIIa is able to form intermolecular gamma-glutamyl-epsilon-lysine cross-links between side chains of fibrin molecules and between other substrates. FXIII is found in plasma and in platelets. The enzyme exists in plasma as a tetrameric zymogen consisting of two alpha-subunits and two beta-subunits (designated $a_2b_2$) and in platelets as a zymogen consisting of two alpha-subunits (designated $a_2$-dimer).

Both zymogens are activated by thrombin and Ca2+. Calcium is being released from the platelets on the aggregation at the site of injury. Thrombin cleaves off the 1-37 N-terminal amino acid residues (of $a_2$-dimer). In case of the $a_2b_2$-zymogen, the beta-subunits are then dissociated from the activated alpha-subunits. Calcium binds equally well to the zymogen and to the thrombin modified molecule. Following the thrombin and calcium activation the active centre cysteine on the alpha chain is exposed and the fully activated enzyme is formed. Subjects with severe thrombocytopenia have been found to have low plasma levels of FXIII.

It is well known that subjects who bleed excessively in association with surgery or major trauma and need blood transfusions develop more complications than those who do not experience any bleeding. However, also moderate bleedings requiring the administration of human blood or blood products (platelets, leukocytes, plasma-derived concentrates for the treatment of coagulation defects, etc.) may lead to complications associated with the risk of transferring human viruses (hepatitis, HIV, parvovirus, and other, by now unknown viruses). Extensive bleedings requiring massive blood transfusions may lead to the development of multiple organ failure including impaired lung and kidney function. Once a subject has developed these serious complications a cascade of events involving a number of cytokines and inflammatory reactions is started making any treatment extremely difficult and unfortunately often unsuccessful. Therefore a major goal in surgery as well as in the treatment of major tissue damage is to avoid or minimise the bleeding

To avoid or minimise such bleeding it is of importance to ensure the formation of stable and solid haemostatic plugs that are not easily dissolved by fibrinolytic enzymes. Furthermore, it is of importance to ensure quick and effective formation of such plugs or clots.

Japanese patent application No. 2-167234 A concerns an adhesive for bio-tissue characterized by containing fibrinogen, prothrombin, blood coagulation factor VII, blood coagulation factor IX, blood coagulation factor X, blood coagulation factor XIII, antithrombin, a proteinase inhibitor, and calcium ion.

Japanese patent application No. 59-116213A concerns an aerosol composition for use as a tissue glue containing a blood coagulant as an active component. The blood coagulant may be selected from blood coagulation factors I, II, III, IV, V, VII, VIII, IX, X, XI, XII, and XIII, prekallikrein, high polymer kininogen and thrombin. A combination of F XIII and thrombin is preferred.

WO 93/12813 (ZymoGenetics) concerns the use of FXIII for reducing perioperative blood loss in a subject undergoing surgery. The composition may also comprise aprotinin. The FXIII is administered to the subject as a bolus injection, typically one day prior to surgery.

European Patent No. 225.160 (Novo Nordisk) concerns compositions of FVIIa and methods for the treatment of bleeding disorders not caused by clotting factor defects or clotting factor inhibitors.

European Patent No. 82.182 (Baxter Travenol Lab.) concerns a composition of factor VIIa for use in counteracting deficiencies of blood clotting factors or the effects of inhibitors to blood clotting factors in a subject.

International Patent Publication No. WO 93/06855 (Novo Nordisk) concerns the topical application of FVIIa.

Kjalke et al, Thrombosis and Haemostasis, 1999 (Suppl), 095 1 concerns the administration of extra exogenous FVIIa and the effect on the formation of thrombin on the activated platelet surface in a model system mimicking hemophilia A or B conditions

There remains a need in the art for an improved, reliable and widely applicable method of enhancing coagulation, quickly forming stable haemostatic plugs and achieving full haemostasis in subjects, in particular in subjects having an impaired thrombin generation. There is also a need for a method wherein the amount of FVIIa needed for achieving full haemostasis is lowered.

SUMMARY OF THE INVENTION

One object of the present invention is to provide compositions, for use in the treatment or prophylaxis of bleeding episodes and coagulation disorders.

The present inventors have shown that a combination of a factor VIIa and a factor XIII can reduce the clotting time of normal human plasma more effectively than either factor VIIa or factor XIII alone. It has also been shown that a combination of a factor VIIa and a factor XIII can increase the firmness of the clot more effectively than either factor VIIa or factor XIII alone. By combining a factor VIIa at a concentration where no further increase in clot firmness was observed with a factor XIII, also at a concentration where no further increase in clot firmness was observed, it was unexpectedly shown that a further increase in clot firmness was obtained. It has also been shown that combination of a factor VIIa and a factor XIII can prolong the in vitro clot lysis time in normal human plasma more effectively than either factor VIIa or factor XIII inhibitor alone. Thus, by enhancing coagulation a more effective treatment of bleeding in subjects can be obtained. Moreover, patients can be treated with relatively lower concentrations of factor VIIa, thus, reducing the relatively high costs in connection with conventional treatment with factor VIIa alone.

In a first aspect, a pharmaceutical composition comprising a factor VIIa and a factor XIII and, optionally, a pharmaceutically acceptable carrier is described.

In another aspect, a pharmaceutical composition comprising a factor VIIa and a factor XIII as the sole active agents and, optionally, a pharmaceutically acceptable carrier is described.

In another aspect, a pharmaceutical composition formulated for intravenous administration comprising a factor VIIa and a factor XIII, optionally, a pharmaceutically acceptable carrier is described.

In another aspect, a pharmaceutical composition formulated for intravenous administration comprising a factor VIIa and a factor XIII as the sole active agents and, optionally, a pharmaceutically acceptable carrier is described.

In one embodiment, the factor VIIa is recombinant factor VIIa and the factor XIII is recombinant factor XIII.

In one embodiment of the invention, the factor VIIa is recombinant factor VIIa. In a further embodiment the factor VIIa is recombinant human factor VIIa. In a further embodiment the factor VIIa is a factor VIIa variant.

In one embodiment, the factor VIIa variants are amino acid sequence variants having no more than 20 amino acids replaced, deleted or inserted compared to wild-type factor VIIa (i.e., a polypeptide having the amino acid sequence disclosed in U.S. Patent No. 4,784,950), In another embodiment, the factor VIIa variants have no more than 15 amino acids replaced, deleted or inserted; in another embodiment, the factor VIIa variants have no more than 10 amino acids replaced, deleted or inserted; in another embodiment, the factor VIIa variants have no more than 8 amino acids replaced, deleted or inserted; in another embodiment, the factor VIIa variants have no more than 6 amino acids replaced, deleted or inserted; in another embodiment, the factor VIIa variants have no more than 5 amino acids replaced, deleted or inserted; in another embodiment, the factor VIIa variants have no more than 3 amino acids replaced, deleted or inserted compared to wild-type factor VIIa. In one embodiment, the factor VIIa variants are selected from the list of [L305V]-FVIIa, [L305V/M306D/D309S]-FVIIa, [L305I]-FVIIa, [L305T]-FVIIa, [F374P]-FVIIa, [V158T/M298Q]-FVIIa, [V158D/E296V/M298Q]-FVIIa and [K337A]-FVIIa.

In one embodiment, the factor XIII is FXIII a2b2. In a further embodiment the factor XIII is FXIII a2. In a further embodiment the factor XIII is activated factor XIII (FXIIIa). In one embodiment the factor XIII is a factor XIII variant. In one embodiment the factor XIII is human factor XIII. In one embodiment the factor XIII is recombinant factor XIII. In one embodiment the factor XIII is recombinant human factor XIII. In one embodiment, the factor XIII is human a2-dimer.

In one aspect, the composition further contains a TFPI inhibitor. In another aspect, the composition further contains a factor VIII. In another aspect, the composition further contains a factor VIII and a TFPI inhibitor.

In one embodiment, the composition further comprises an inhibitor of the fibrinolytic system, e.g., aprotinin, ε -aminocaproic acid or tranexamic acid.

The invention relates to the use of a factor VIIa in combination with a factor XIII for the manufacture of a medicament for treating bleeding episodes.

In another aspect, the invention relates to the use of a factor VIIa in combination with a factor XIII for the manufacture of a medicament for reducing clotting time in a subject.

In another aspect, the invention relates to the use of a factor VIIa in combination with a factor XIII for the manufacture of a medicament for prolonging the clot lysis time in mammalian plasma.

In another aspect, the invention relates to the use of a factor VIIa in combination with a factor XIII for the manufacture of a medicament for increasing clot strength in mammalian plasma.

In another aspect, the invention relates to the use of a factor VIIa in combination with a factor XIII for the manufacture of a medicament for enhancing fibrin clot formation in mammalian plasma.

In one embodiment, the mammalian plasma is human plasma. In another embodiment, the mammalian plasma is normal plasma; in another embodiment, the plasma is normal human plasma; in one embodiment, the plasma is plasma from a subject having an impaired thrombin generation. In one embodiment, the plasma is from a subject having a lowered concentration of fibrinogen.

In one embodiment, the factor VIIa and the factor XIII prolong the in vitro clot lysis time in normal human plasma.

In one embodiment of the methods of the invention, the factor VIIa and the factor XIII are the sole active agents to be administered to the subject. In another embodiment of the invention, the pharmaceutical composition comprises a factor VIIa and a factor XIII as the sole active agents.

In one embodiment of the invention, the factor VIIa and the factor XIII are to be administered simultaneously and in one-dosage form. In another embodiment, the factor VIIa and the factor XIII is to be administered sequentially. In a further embodiment, the factor VIIa and the factor XIII is administered within about 1-2 hours of each other, for example within 30 minutes of each other, for instance within 10 minutes of each other, e.g., in the form of a kit comprising a factor VIIa in a first unit dosage form and a factor XIII in a second unit dosage form.

In one embodiment, the effective amount of a factor VIIa is from 0.05 mg/day to 500 mg/day (70-kg subject). In one embodiment, the effective amount of a factor XIII is from 0.05 mg/day to 500 mg/day (70-kg subject).

In one embodiment of the present invention, the pharmaceutical composition (when in single-preparation form) consists essentially of a factor VIIa and a factor XIII, and, optionally, at least one pharmaceutical acceptable excipient or carrier, and/or a stabiliser, and/or a detergent, and/or a neutral salt, and/or an antioxidant, and/or a preservative, and/or a protease inhibitor.

In another embodiment of the present invention, the pharmaceutical composition (when in single-preparation form) consists essentially of a factor VIIa and a factor XIII, and, optionally, at least one pharmaceutical acceptable excipient or carrier, and/or a stabiliser, and/or a detergent, and/or a neutral salt, and/or an antioxidant, and/or a preservative, and/or a protease inhibitor, and/or a TFPI-inhibitor.

In another embodiment of the present invention, the pharmaceutical composition (when in single-preparation form) consists essentially of a factor VIIa and a factor XIII, and, optionally, at least one pharmaceutical acceptable excipient or carrier, and/or a stabiliser, and/or a detergent, and/or a neutral salt, and/or an antioxidant, and/or a preservative, and/or a protease inhibitor, and/or a TFPI-inhibitor, and/or a factor VIII.

In another embodiment, the pharmaceutical composition (when in form of a kit) consists of a first unit dosage form consisting essentially of a factor VIIa and, optionally, at least one pharmaceutical acceptable excipient or carrier, and/or a stabiliser, and/or a detergent, and/or a neutral salt, and/or an antioxidant, and/or a preservative, and/or a protease inhibitor, and a second unit dosage form consisting essentially of a factor XIII, and, optionally, at least one pharmaceutical acceptable excipients or carrier, and/or a stabiliser, and/or a detergent, and/or a neutral salt, and/or an antioxidant, and/or a preservative, and/or a protease inhibitor.

In another embodiment, the pharmaceutical composition (when in form of a kit) consists of a first unit dosage form consisting essentially of a factor VIIa and, optionally, at least one pharmaceutical acceptable excipient or carrier, and/or a stabiliser, and/or a detergent, and/or a neutral salt, and/or an antioxidant, and/or a preservative, and/or a protease inhibitor, and/or a TFPI-inhibitor; and a second unit dosage form consisting essentially of a factor XIII, and, optionally, at least one pharmaceutical acceptable excipients or carrier, and/or a stabiliser, and/or a detergent, and/or a neutral salt, and/or an antioxidant,and/or a preservative, and/or a protease inhibitor, and/or a TFPI-inhibitor, and/or a factor VIII.

In a further embodiment, the subject is a human; in another embodiment, the subject has an impaired thrombin generation; in one embodiment, the subject has a lowered plasma concentration of fibrinogen (e.g., a multi-transfused subject).

In a further aspect, the composition further contains a factor VIII. In one embodiment, the factor VIII is an activated factor VIII (factor VIIIa). In a further embodiment the factor VIII is a recombinant factor VIIIa. In a further embodiment the factor VIII is recombinant human factor VIIIa.

In a further aspect, the composition further comprises an inhibitor of the fibrinolytic system, e.g., aprotinin, ε-aminocaproic acid or tranexamic acid.

LIST OF FIGURES

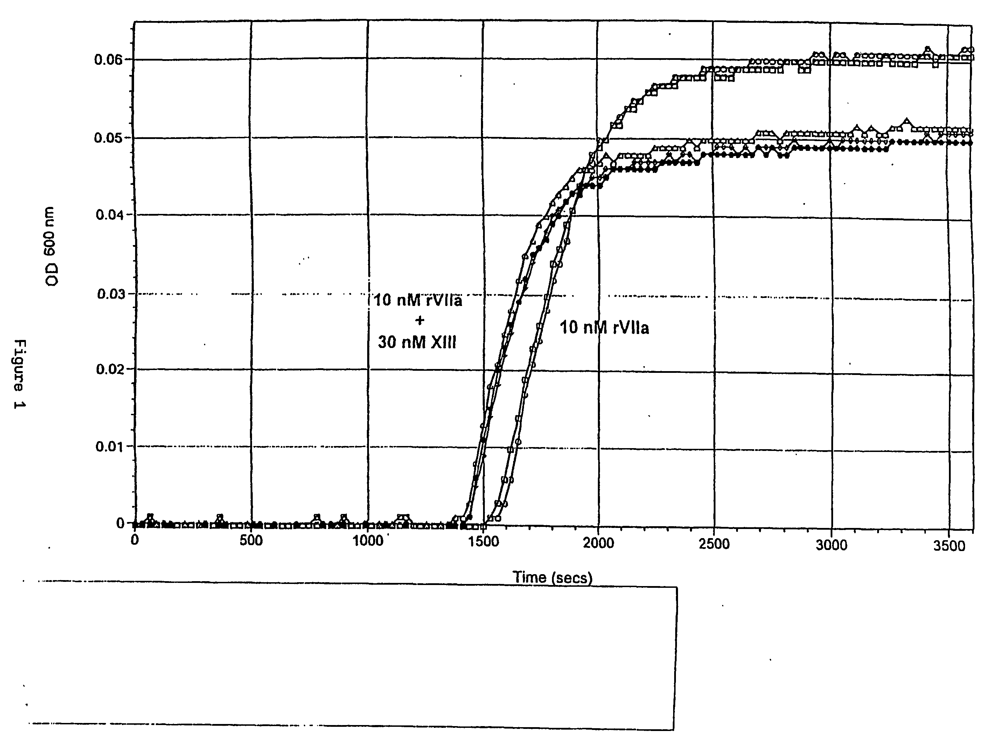

Figure 1 shows that the spontaneous clot formation in citrated normal human plasma (NHP) diluted to 1/10 in buffer containing 20 nM Hepes, 150 mM NaCl, and 5 mM $CaCl_2$, pH 7.4 in a micro titer well (total volume 250 μl) was obtained at about 2500-3000 sec. Fibrin clot formation was monitored by the increase in optical density at 600 nm in a Specramax™ 340. Molecular Devices, Sunnyvale CA. Fig. 1 shows that 10 nM recombinant factor VIIa (rFVIIa) from Novo Nordisk A/S Bagsværd, Denmark shortened the clotting time to 1600 sec (n = 2). Further shortening of the clotting time was obtained when 30 nM factor XIII (FXIII) from American Diagnostica inc, Greenwich, CT was added together with 10 nM rFVIIa (n = 3). The clot formed in the presence of FXIII was more transparent (lower maximal OD) than in its absence indicating that the addition of FXIII resulted in a more fine-meshed fibrin gel structure with thinner fibres.

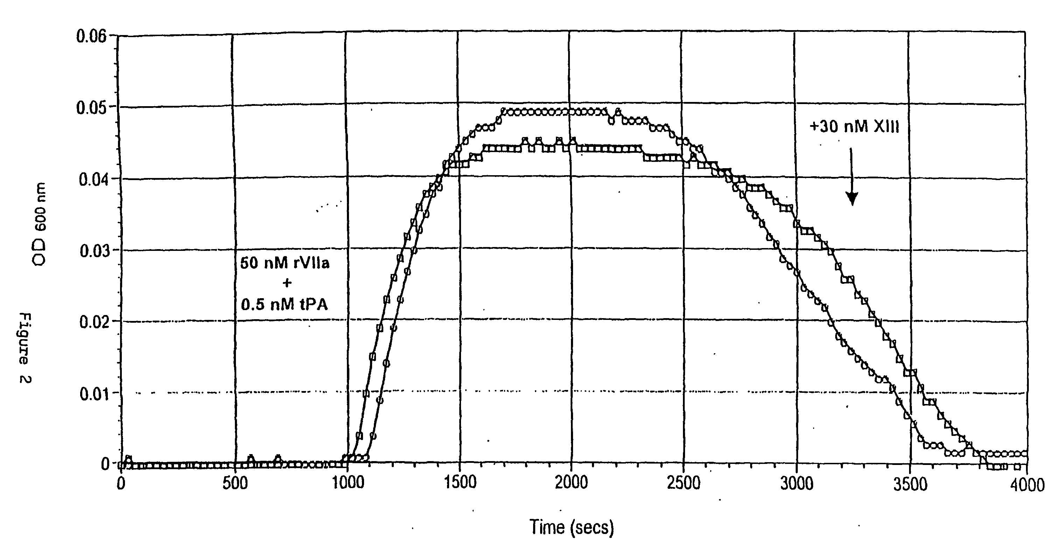

Figure 2 shows that supplementary FXIII (30 nM) prolongs the fibrin clot lysis time of clots formed in the presence of rFVIIa and tissue plasminogen activator (tPA). Clot formation was induced in the presence or absence of 30 nM XIII by addition of 25 μl NHP to 225 μl 20 nM Hepes, 150 mM NaCl, 5 mM $CaCl_2$, pH 7.4 containing 50 nM rFVIIa and 0.5 nM recombinant tPA from Novo Nordisk A/S Bagsværd, Denmark. Clot formation and subsequent clot lysis induced by tPA-mediated plasminogen activation was monitored by a Spectramax® 340 at 600 nm as the increase in $OD_{600\ nm}$ followed by reversion of the trace to the basal level. Fig. 2 shows that the clot lysis time under these conditions was significantly prolonged by the presence of FXIII.

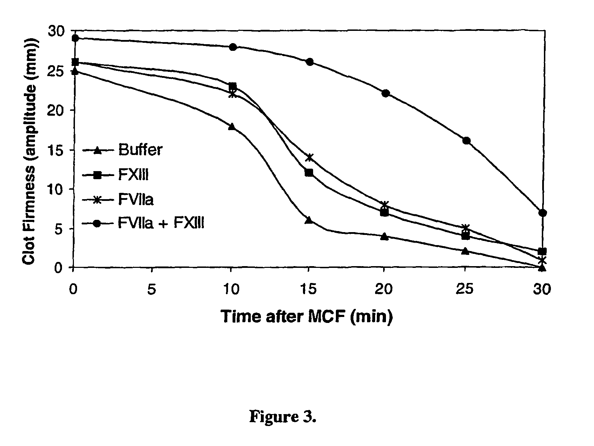

Figure 3 shows the effect of rFVIIa and FXIII on Maximal Clot Firmness (MCF) as well as clot resistance to t-PA mediated lysis. Prior to rFVIIa and/or FXIII addition the MCF obtained was 25 mm and the time required for half the clot to be lysed was 12.3 minutes (Fig. 3). Addition of increasing concentrations of FXIII (0 - 40 nM) did not alter MCF; however, a dose-dependent prolongation of the half-clot lysis was observed, optimal at 30 nM FXIII (half-clot lysis time: 14,3 min, Fig. 3). Similarly, rFVIIa (1 nM) addition resulted in clot protection from t-PA-mediated fibrinolysis (half-clot lysis time; 16.4 min) without any effect on MCF (Fig. 3). However, upon addition of rFVIIa (1 nM) together with FXIII (30 nM) an increase in the MCF (29 mm), as well as a profound protection from fibrinolysis (half-clot lysis time; 27.1 min) was observed (Fig. 3). Taken together, these results demonstrate that rFVIIa and FXIII addition to plasma in a synergistic fashion improve clot mechanical strength and resistance to t-PA mediated fibrinolysis.

DETAILED DESCRIPTION OF THIS INVENTION

The present invention provides a composition comprising a combination of a factor VIIa and a factor XIII. The invention also provides a composition comprising a combination of a factor VIIa and a factor XIII as the sole active ingredients. The composition may be in the form of a single composition or it may be in the form of a multi-component kit. The present compositions are useful as therapeutic and prophylactic procoagulant and fibrin clot-stabilizing agents and form quick fibrin clots in mammals, including primates such as humans.

Whenever, a first or second or third, etc., unit dose is mentioned throughout this specification this does not indicate the preferred order of administration, but is merely done for convenience purposes.

The present invention further provides means for treating (including prophylactically treating or preventing) bleeding episodes in a subject, including a human being, e.g., due to trauma or surgery, or in subjects lacking or having defective blood coagulation factors FIX or FVIII or platelets.

It has now been found that a combination of a factor VIIa and a factor XIIIa is an advantageous product ensuring the formation of solid, stable and quick haemostatic plugs.

The full thrombin generation is necessary for a solid, stabile haemostatic plug to be formed. The fibrin structure of such a plug is dependent on both the amount of thrombin formed and the rate of the initial thrombin generation. In the presence of an impaired thrombin generation a porous fibrin plug, which is highly permeable, is being formed. The fibrinolytic enzymes normally present on the fibrin surface easily dissolve such a fibrin plug. The formation of a stable fibrin plug is also dependent on the presence of factor XIIIa, which is being activated by thrombin and therefore also dependent on the full thrombin generation. Furthermore, the recently described thrombin activatable fibrinolytic inhibitor, TAFI, requires rather high thrombin amounts for its activation. In the presence of a not fully adequate thrombin formation the TAFI may therefore not be activated resulting in the formation of a haemostatic plug, which is easier than normally dissolved by the normal fibrinolytic activity.

By increasing the thrombin generation factor VIIa provides the basis for a full factor XIII activation, which is of the uttermost importance for the formation of a fully stabilised haemostatic plug and thereby for the maintenance of haemostasis. In situations with lowered number of platelets, thrombocytopenia, a faster thrombin generation is initiated by the administration of exogenous extra factor VIIa. However, the total thrombin generation is not normalised by factor VIIa even in high concentrations.

By combining a factor VIIa and a factor XIII, in particular the factor XIII alpha chain (a2-dimer), a full activation of factor XIII is facilitated which enhances the haemostatic effect of factor VIIa.

Furthermore, in subjects with lowered plasma concentrations of fibrinogen (multi-transfused subjects as a consequence of multiple trauma or extensive surgery) full factor XIII activation does not occur. A more effective haemostasis is then obtained by the administration of a combination of a factor VIIa and a factor XIII.

Another way to increase the stability of fibrin haemostatic plugs is to ensure full presence of factor XIIIa (activated factor XIII).

Subjects with thrombocytopenia have an impaired thrombin generation as well as a defective stabilization of the fibrin plugs resulting in haemostatic plugs prone to premature dissolution. Furthermore, subjects subjected to major trauma or organ damage and who, as a consequence, have obtained frequent blood transfusions often have lowered platelet counts as well as lowered levels of fibrinogen, factor VIII, and other coagulation proteins. These subjects experience an impaired (or lowered) thrombin generation. In addition, their lowered fibrinogen level interfere negatively with the activation of factor XIII. These subjects, therefore, have a defective, or less efficient, haemostasis leading to the formation of fibrin plugs which are easily and prematurely dissolved by proteolytic enzymes, such enzymes in addition being extensively released in situations characterized by extensive trauma and organ damage.

In order to facilitate the formation of fully stabilized plugs with full capacity to maintain haemostasis in a subject, a composition according to the invention is administered. This composition is especially beneficial in subjects with a lowered number of platelets and in subjects with lowered plasma levels of fibrinogen and/or other coagulation proteins.

In the presence of a factor XIII lower concentrations of factor VIIa may be sufficient to ensure a sufficient haemostasis.

As stated above, factor XIII exists in plasma as a tetrameric zymogen consisting of two alpha-subunits and two beta-subunits (designated a2 b2), but is found in other tissue (e.g., platelets) as an a2-dimer. Either of these zymogen forms, or activated factor XIII (factor XIIIa), may be used within the present invention, as well as genetically engineered variants of factor XIII that retain their characteristic cross-linking activity. In one embodiment, the factor XIII is human factor XIII; in another embodiment, the factor XIII is human a2-dimer; in yet another embodiment, the factor XIII Is activated human factor XIIIa.

The factor XIII and factor VIIa used in the present invention may be purified from blood or produced by recombinant means. It is evident that the practice of the methods described herein is independent of how the purified factor XIII and factor VIIa are derived and, therefore, the present invention is contemplated to cover use of any factor XIII and factor VIIa preparation suitable for use herein. Preferred are human factor VIIa and human factor XIII. Also genetically engineered variants of factor VIIa and factor XIII retaining their characteristic haemostasis-related activity may be used in the present inventions. Fragments of factor VIIa or factor XIII or factor VIIa- or factor XIII-variants retaining their characteristic haemostasis-related activity may also be used in the present inventions. The haemostasis-related activity of a factor VIIa may, for example, be measured using the factor VIIa-activity assay described in the present specification. The haemostasis-related activity of a factor XIII may, for example, be measured using the factor XIII-activity assay described in the present specification.

Non-limiting examples of factor VII variants having substantially the same or better biological activity compared to wild-type factor VIIa include, but are not limited to, those described in Danish Patent Applications Nos. PA 2000 00734, PA 2000 01360, PA 2000 01361, and PA 2001 00477. Non-limiting examples include [L305V]-FVIIa, [L305V/M306D/D309S]-FVIIa, [L305I]-FVIIa, [L305T]-FVIIa, [F374P]-FVIIa, [V158T/M298Q]-FVIIa, [V158D/E296V/M298Q]-FVIIa and [K337A]-FVIIa.

In the present context the three-letter or one-letter indications of the amino acids have been used in their conventional meaning as indicated in table 1. Unless indicated explicitly, the amino acids mentioned herein are L-amino acids. It is to be understood, that the first letter in, for example, K337 represent the amino acid naturally present at the indicated position wild-type factor VII, and that, for example, [K337A]-FVIIa designates the FVII-variant wherein the amino acid represented by the one-letter code K naturally present in the indicated position is replaced by the amino acid represented by the one-letter code A.

Table 1: Abbreviations for amino acids:

| Amino acid | Tree-letter code | One-letter code |
|---|---|---|
| Glycine | Gly | G |
| Proline | Pro | P |
| Alanine | Ala | A |
| Valine | Val | V |
| Leucine | Leu | L |
| Isoleucine | Ile | I |
| Methionine | Met | M |
| Cysteine | Cys | C |
| Phenylalanine | Phe | F |
| Tyrosine | Tyr | Y |
| Tryptophan | Trp | W |
| Histidine | His | H |
| Lysine | Lys | K |
| Arginine | Arg | R |
| Glutamine | Gln | Q |
| Asparagine | Asn | N |
| Glutamic Acid | Glu | E |
| Aspartic Acid | Asp | D |

The term "factor VIIa" or "FVIIa" may be used interchangeably. The term factor VIIa includes zymogen factor VII (single-chain factor VII). The term "factor XIII" or "FXIII" may be used interchangeably. The term "factor VIII" or "FVIII" may be used interchangeably.

It will be apparent to those skilled in the art that substitutions can be made outside the regions critical to the function of the factor VIIa or factor XIII-molecule and still result in an active polypeptide. Amino acid residues essential to the activity of the factor VIIa or factor XIII-polypeptide, and therefore preferably not subject to substitution, may be identified according to procedures known in the art, such as site-directed mutagenesis or alanine-scanning mutagenesis (see, e.g., Cunningham and Wells, 1989, *Science* 244: 1081-1085). In the latter technique, mutations are introduced at every positively charged residue in the molecule, and the resultant mutant molecules are tested for coagulant, respectively cross-linking activity to identify amino acid residues that are critical to the activity of the molecule. Sites of substrate-enzyme interaction can also be determined by analysis of the three-dimensional structure as determined by such techniques as nuclear magnetic resonance analysis, crystallography or photoaffinity labelling (see, *e.g.,* de Vos *et al*., 1992, *Science* 255: 306-312; Smith *et al*., 1992, *Journal of Molecular Biology* 224: 899-904; Wlodaver *et al*., 1992, *FEBS Letters* 309: 59-64).

The introduction of a mutation into the nucleic acid sequence to exchange one nucleotide for another nucleotide may be accomplished by site-directed mutagenesis using any of the methods known in the art. Particularly useful is the procedure that utilizes a super coiled, double stranded DNA vector with an insert of interest and two synthetic primers containing the desired mutation. The oligonucleotide primers, each complementary to opposite strands of the vector, extend during temperature cycling by means of *Pfu* DNA polymerase. On incorporation of the primers, a mutated plasmid containing staggered nicks is generated. Following temperature cycling, the product is treated with *Dpn*I, which is specific for methylated and hemimethylated DNA to digest the parental DNA template and to select for mutation-containing synthesized DNA. Other procedures known in the art for creating, identifying and isolating variants may also be used, such as, for example, gene shuffling or phage display techniques.

The term "factor VIII" or "FVIII" includes activated factor VIII (designated factor VIIIa), variants and truncated forms of factor VIII retaining their characteristic coagulant activity. In one embodiment, the factor VIII is human factor VIII.

The term "TFPI inhibitor" means compounds inhibiting the anti-coagulative activity of TFPI (tissue factor pathway inhibitor). The term includes compounds such as those disclosed in European Patent No. 558 529, WO 96/28153 and US 5,622,988. "TFPI" and "EPI" (extrinsic pathway inhibitor) may be used interchangeably.

Within the present invention an "effective amount" of a factor VIIa and an "effective amount" of a factor XIII is defined as the amount of a factor VIIa and a factor XIII sufficient to prevent or reduce bleeding or blood loss, so as to cure, alleviate or partially arrest the disease and its complications.

The amount of a factor VIIa and the amount of a factor XIII administered according to the present invention may vary from a ratio of about 1:100 to about 100:1 (μg factor VIIa : μg factor XIII).

In this context, "subjects with an impaired thrombin generation" means subjects who cannot generate a full thrombin burst on the activated platelet surface and includes subjects having a generation of thrombin less that the thrombin-generation in subjects having a fully functioning, normal haemostatic system, including a normal amount and function of coagulation factors, platelets and fibrinogen, and includes subjects lacking FIX and/or FVIII (haemophilia A and B) or having defective FIX and/or FVIII or having inhibitors against FIX and/or FVIII; subjects lacking FXI; subjects with a lowered number of platelets or platelets with a defective function (e.g., thrombocytopenia or thrombasthenia Glanzmann or subjects with excessive bleeds); and subjects having lowered levels of prothrombin, FX or FVII.

Subjects with lowered plasma concentrations of fibrinogen (e.g., multitransfused subjects as a consequence of multiple trauma or extensive surgery) do also suffer from the formation of looser and unstable fibrin plugs which are easily dissolved.

The term "full haemostasis" means the formation of a stable and solid fibrin clot or plug at the site of injury which effectively stops the bleeding and which is not readily dissolved by the fibrinolytic system.

The term "activity of factor VIIa" or "factor VIIa-activity" means the ability to generate thrombin; the term also includes the ability to generate thrombin on the surface of activated platelets in the absence of tissue factor.

The term "enhancement of the normal haemostatic system" means an enhancement of the ability to generate thrombin.

As used herein the term "bleeding disorder" reflects any defect, congenital, acquired or induced, of cellular or molecular origin that is manifested in bleedings. Examples are clotting factor deficiencies (e.g. haemophilia A and B or deficiency of coagulation factors XI or VII), clotting factor inhibitors, defective platelet function, thrombocytopenia or von Willebrand's disease.

The term "bleeding episodes" is meant to include uncontrolled and excessive bleeding which is a major problem both in connection with surgery and other forms of tissue damage. Uncontrolled and excessive bleeding may occur in subjects having a basically normal coagulation system (these subjects do however develop a coagulopathy as a result of the bleeding — dilution of coagulation proteins, increased fibrinolysis and lowered platelets due to a dilution effect of the bleeding) and subjects having coagulation or bleeding disorders. Clotting factor deficiencies (haemophilia A and B, deficiency of coagulation factors XI or VII) or clotting factor inhibitors may be the cause of bleeding disorders. Excessive bleedings also occur in subjects with a normally functioning blood clotting cascade (no clotting factor deficiencies or -inhibitors against any of the coagulation factors) and may be caused by a defective platelet function, thrombocytopenia or von Willebrand's disease. In such cases, the bleedings may be likened to those bleedings caused by haemophilia because the haemostatic system, as in haemophilia, lacks or has abnormal essential clotting "compounds" (such as platelets or von Willebrand factor protein) that causes major bleedings. In subjects who experience extensive tissue damage in association with surgery or vast trauma, the normal haemostatic mechanism may be overwhelmed by the demand of immediate haemostasis and they may develop bleeding in spite of a basically (pre-trauma) normal haemostatic mechanism. Achieving satisfactory haemostasis also is a problem when bleedings occur in organs such as the brain, inner ear region and eyes with limited possibility for surgical haemostasis. The same problem may arise in the process of taking biopsies from various organs (liver, lung, tumour tissue, gastrointestinal tract) as well as in laparoscopic surgery. Common for all these situations is the difficulty to provide haemostasis by surgical techniques (sutures, clips, etc.) which also is the case when bleeding is diffuse (haemorrhagic gastritis and profuse uterine bleeding). Acute and profuse bleedings may also occur in subjects on anticoagulant therapy in whom a defective haemostasis has been induced by the therapy given. Such subjects may need surgical interventions in case the anticoagulant effect has to be counteracted rapidly. Radical retropubic prostatectomy is a commonly performed procedure for subjects with localized prostate cancer. The operation is frequently complicated by significant and sometimes massive blood loss. The considerable blood loss during prostatectomy is mainly related to the complicated anatomical situation, with various densely vascularized sites that are not easily accessible for surgical haemostasis, and which may result in diffuse bleeding from a large area. Another situation that may cause problems in the case of unsatisfactory haemostasis is when subjects with a normal haemostatic mechanism are given anticoagulant therapy to prevent thromboembolic disease. Such therapy may include heparin, other forms of proteoglycans, warfarin or other forms of vitamin K-antagonists as well as aspirin and other platelet aggregation inhibitors.

In one embodiment of the invention, the bleeding is associated with haemophilia. In another embodiment, the bleeding is associated with haemophilia with aquired inhibitors. In another embodiment, the bleeding is associated with thrombocytopenia. In another embodiment, the bleeding is associated with von Willebrand's disease. In another embodiment, the bleeding is associated with severe tissue damage. In another embodiment, the bleeding is associated with severe trauma. In another embodiment, the bleeding is associated with surgery. In another embodiment, the bleeding is associated with laparoscopic surgery. In another embodiment, the bleeding is associated with haemorrhagic gastritis. In another embodiment, the bleeding is profuse uterine bleeding. In another embodiment, the bleeding is occurring in organs with a limited possibility for mechanical haemostasis. In another embodiment, the bleeding is occurring in the brain, inner ear region or eyes. In another embodiment, the bleeding is associated with the process of taking biopsies. In another embodiment, the bleeding is associated with anticoagulant therapy.

The composition according to the invention may further comprise a TFPI-inhibitor. Such a composition should preferable be administered to subjects having haemophilia A or B.

The composition according to the invention may further comprise a factor VIII. Such a composition should preferably be administered to subjects who do not have inhibitors to factor VIII.

In this context, the term "treatment" is meant to include both prevention of an expected bleeding, such as, for example, in surgery, and regulation of an already occurring bleeding, such as, for example, in haemophilia or in trauma, with the purpose of inhibiting or minimising the bleeding. Prophylactic administration of a factor VIIa and a factor XIII is thus included in the term "treatment".

The term "subject" as used herein is intended to mean any animal, in particular mammals, such as humans, and may, where appropriate, be used interchangeably with the term "patient".

Abbreviations

| | |
|---|---|
| TF | tissue factor |
| FVII | factor VII in its single-chain, unactivated form |
| FVIIa | factor VII in its activated form |
| rFVIIa | recombinant factor VII in its activated form |
| FXIII | factor XIII in its zymogenic, unactivated form |
| FXIIIa | factor XIII in its activated form |
| rFXIII | recombinant FXIII |
| rFXIIIa | recombinant FXIIIa |
| a2 | alpha- or a-chain of FXIII or rFXIII |
| b2 | beta- or b-chain of FXIII or rFXIII |
| FXIIIa2 | dimeric form of FXIII containing two a-chains |
| FXIIIa2b2 | tetrameric form of FXIII containing two a- and two b-chains |
| FVIII | factor VIII in its zymogenic, unactivated form |
| rFVIII | recombinant FVIII |
| FVIIIa | factor VIII in its activated form |
| rFVIIIa | recombinant FVIIIa |
| TFPI | tissue factor pathway inhibitor |

Preparation of compounds

Human purified factor VIIa suitable for use in the present invention is preferably made by DNA recombinant technology, e.g. as described by Hagen et al., Proc.Natl.Acad.Sci. USA 83: 2412-2416, 1986 or as described in European Patent No. 200.421 (ZymoGenetics, Inc.). factor VIIa produced by recombinant technology may be authentic factor VIIa or a more or less modified factor VIIa provided that such a factor VIIa has substantially the same biological activity for blood coagulation as authentic factor VIIa (wild-type factor VIIa). Such a modified factor VIIa may be produced by modifying the nucleic acid sequence encoding wild-type factor VII either by altering the amino acid codons or by removal of some of the amino acid codons in the nucleic acid encoding the natural factor VII by known means, e.g. by site-specific mutagenesis.

Factor VII may also be produced by the methods described by Broze and Majerus, J.Biol.Chem. 255 (4): 1242-1247, 1980 and Hedner and Kisiel, J.Clin.Invest. 71: 1836-1841, 1983. These methods yield factor VII without detectable amounts of other blood coagulation factors. An even further purified factor VII preparation may be obtained by including an additional gel filtration as the final purification step. factor VII is then converted into activated factor VIIa by known means, e.g. by several different plasma proteins, such as factor XIIa, IX a or Xa. Alternatively, as described by Bjoem et al. (Research Disclosure, 269 September 1986, pp. 564-565), factor VII may be activated by passing it through an ion-exchange chromatography column, such as Mono Q® (Pharmacia fine Chemicals) or the like.

Factor XIII for use within the present invention may be prepared from plasma according to known methods, such as those disclosed by Cooke and Holbrook (Biochem. J. 141: 79-84, 1974) and Curtis and Lorand (Methods Enzymol. 45: 177-191, 1976), incorporated herein by reference. The a2 dimer form of factor XIII may be prepared from placenta as disclosed in U.S. Pat. Nos. 3,904,751; 3,931,399; 4,597,899 and 4,285,933, incorporated herein by reference. It is preferred, however, to use recombinant factor XIII so as to avoid to the use of blood- or tissue-derived products that carry a risk of disease transmission. Methods for preparing recombinant factor XIII are known in the art. See, for example, Davie et al., EP 268,772; Grundmann et al., AU-A-69896/87; Bishop et al., Biochemistry 1990, 29: 1861-1869; Board et al., Thromb. Haemost. 1990, 63: 235-240; Jagadeeswaran et al., Gene 1990, 86: 279-283; and Bröker et al., FEBS Lett. 1989, 248: 105-110, which are incorporated herein by reference in their entirety. Within one embodiment, the factor XIII a2 dimer is prepared cytoplasmically in the yeast Saccharomyces cerevisiae as disclosed in copending U.S. patent application Ser. No. 07/741,263, incorporated herein by reference in its entirety). The cells are harvested and lysed, and a cleared lysate is prepared. The lysate is fractionated by anion exchange chromatography at neutral to slightly alkaline pH using a column of derivatized agarose, such as DEAE Fast-Flow Sepharose.TM. (Pharmacia) or the like. factor XIII is then precipitated from the column eluate by concentrating the eluate and adjusting the pH to 5.2-5.5, such as by diafiltration against ammonium succinate buffer. The precipitate is then dissolved and further purified using conventional chromatographic techniques, such as gel filtration and hydrophobic interaction chromatography.

As will be appreciated by those skilled in the art, it is preferred to use factor XIII and factor VIIa proteins syngeneic with the subject in order to reduce the risk of inducing an immune response. Preparation and characterization of non-human factor XIII has been disclosed by Nakamura et al. (J. Biochem. 78: 1247-1266, 1975). The present invention also encompasses the use of such factor XIII and factor VIIa proteins within veterinary procedures.

Administration and pharmaceutical compositions

For treatment in connection with deliberate interventions, the factor VII and the factor XIII will typically be administered within about 24 hours prior to performing the intervention, and for as much as 7 days or more thereafter. Administration as a coagulant can be by a variety of routes as described herein.

The dose of the factor VII ranges from about 0.05 mg to about 500 mg/day, e.g., from about 1 mg to about 200 mg/day, or, e.g., from about 10 mg to about 175 mg/day for a 70-kg subject as loading and maintenance doses, depending on the weight of the subject, the condition and the severity of the condition.

The dose of the factor XIII ranges from about 0.05 mg to about 500 mg/day, e.g., from about 1 mg to about 200 mg/day, or, e.g., from about 10 mg to about 175 mg/day for a 70-kg subject as loading and maintenance doses, depending on the weight of the subject, the condition and the severity of the condition.

The compositions and kits described herein are useful within human and veterinary medicine; such as, for example, in the treatment or prophylaxis of subjects suffering from bleeding episodes or coagulative disorders. For use within the present invention, the factor VIIa and factor XIII are formulated, optionally with a pharmaceutically acceptable carrier. Preferably, the pharmaceutical compositions are to be administered parenterally, i.e., intravenously, subcutaneously, or intramuscularly, or it may be administered by continuous or pulsatile infusion.

Formulations may further include one or more diluents, emulsifiers, preservatives, buffers, excipients, etc. and may be provided in such forms as liquids, powders, emulsions, controlled release, etc. One skilled in this art may formulate the compositions of the invention an appropriate manner, and in accordance with accepted practices, such as those disclosed in Remington's Pharmaceutical Sciences, Gennaro, ed., Mack Publishing Co., Easton, PA, 1990. The compositions for parenteral administration comprise a factor VII and a factor XIII in combination with, preferably dissolved in, a pharmaceutically acceptable carrier, preferably an aqueous carrier. A variety of aqueous carriers may be used, such as water, buffered water, 0.4% saline, 0.3% glycine and the like. The factor VII variants of the invention can also be formulated into liposome preparations for delivery or targeting to the sites of injury. Liposome preparations are generally described in, e.g., U.S. 4,837,028, U.S. 4,501,728, and U.S. 4,975,282.

A typical pharmaceutical composition for intravenous infusion could be made up to contain 250 ml of sterile Ringer's solution and 10 mg of a factor VIIa and/or a factor XIII. Actual methods for preparing parenterally administrable compositions will be known or apparent to those skilled in the art and are described in more detail in, for example, Remington's Pharmaceutical Sciences, 18th ed., Mack Publishing Company, Easton, PA (1990).

In short, pharmaceutical compositions suitable for use according to the present invention is made by mixing a factor VIIa, or a factor XIII, or a factor VIIa in combination with a factor XIII, preferably in purified form, with suitable adjuvants and a suitable carrier or diluent. Suitable physiological acceptable carriers or diluents include sterile water and saline. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc. Suitable adjuvants also include calcium, proteins (e.g. albumins), or other inert peptides (e.g. glycylglycine) or amino acids (e.g. glycine, or histidine) to stabilise the purified factor VIIa and/or factor XIII. Other physiological acceptable adjuvants are non-reducing sugars, polyalcohols (e.g. sorbitol, mannitol or glycerol), polysaccharides such as low molecular weight dextrins, detergents (e.g. polysorbate) and antioxidants (e.g. bisulfite and ascorbate). The adjuvants are generally present in a concentration of from 0.001 to 4% w/v. The pharmaceutical composition may also contain protease inhibitors, e.g. aprotinin or tranexamic acid, and preserving agents. Furthermore, the preparation may also contain a TFPI-inhibitor and/or factor VIII.

The compositions may be sterilised by conventional, well-known sterilisation techniques. The resulting aqueous solutions may be packaged for use or filtered under aseptic conditions and lyophilised, the lyophilised preparation being combined with a sterile aqueous solution prior to administration.

The concentration of a factor VIIa, a factor XIII, or a factor VIIa in combination with a factor XIII in these formulations can vary widely, i.e., from less than about 0.5% by weight, usually at or at least about 1 % by weight to as much as 15 or 20% by weight and will be selected primarily by fluid volumes, viscosities, etc., in accordance with the particular mode of administration selected.

Administration by injection or infusion, in particular injection, is preferred. Thus, the factor VIIa and the factor XIII are prepared in a form suitable for intravenous administration, such as a preparation that is either a dissolved lyophilised powder or a liquid formulation containing both the factor VIIa and the factor XIII in one dosage form, or a dissolved lyophilised powder or a liquid formulation containing the factor VIIa in one dosage form and dissolved lyophilised powder or a liquid formulation containing the factor XIII in another dosage form.

Local delivery of a factor VIIa and a factor XIII, such as, for example, topical application may be carried out, for example, by means of a spray, perfusion, double balloon catheters, stent, incorporated into vascular grafts or stents, hydrogels used to coat balloon catheters, or other well established methods. For ambulatory subjects requiring daily maintenance levels, the factor VIIa and the factor XIII may be administered by continuous infusion using e.g. a portable pump system. In any event, the pharmaceutical compositions should provide a quantity of a factor VIIa and a factor XIII sufficient to effectively treat the subject.

The combination of a factor VIIa and a factor XIII shows a synergistic effect in an *in vitro* clot firmness- and fibrinolysis time-assay. Moreover, the combination of a factor VIIa and a factor XIII shows a synergistic effect in forming stable fibrin clots, increasing the half-clot lysis time, increasing clot strength and increasing resistance to fibrinolysis.

The compositions containing a factor VII and a factor XIII can be administered for prophylactic and/or therapeutic treatments. In therapeutic applications, compositions are administered to a subject already suffering from a disease, as described above, in an amount sufficient to cure, alleviate or partially arrest the disease and its complications. An amount adequate to accomplish this is defined as an "effective amount" or "therapeutically effective amount". As will be understood by the person skilled in the art, amounts effective for this purpose will depend on the severity of the disease or injury as well as the weight and general state of the subject. It must be kept in mind that the materials of the present invention may generally be employed in serious disease or injury states, that is, life threatening or potentially life threatening situations. In such cases, in view of the minimisation of extraneous substances and general lack of immunogenicity of factor VIIa and factor XIII in humans, it is possible and may be felt desirable by the treating physician to administer a substantial excess of these compositions.

In prophylactic applications, compositions containing a factor VIIa and a factor XIII are administered to a subject susceptible to or otherwise at risk of a disease state or injury to enhance the subject's own coagulative capability. Such an amount is defined to be a "prophylactically effective dose."

Single or multiple administrations of the compositions can be carried out with dose levels and patterns being selected by the treating physician. The compositions may be administered one or more times per day or week. An effective amount of such a pharmaceutical composition is the amount that provides a clinically significant effect against bleeding episodes. Such amounts will depend, in part, on the particular condition to be treated, age, weight, and general health of the subject, and other factors evident to those skilled in the art.

The composition is generally administered in one single dose before the expected bleeding or at the start of the bleeding. It may however also be given in multiple doses, preferably with intervals of 2-4-6-12 hour, depending on the dose given and the condition of the subject.

The composition may be in the form of a single preparation comprising both a factor VIIa and a factor XIII in suitable concentrations. The composition may also be in the form of a kit consisting of a first unit dosage form comprising a factor VIIa and a second unit dosage form comprising a factor XIII and, optionally, one or more further unit dosage forms comprising a factor VIII and/or an TFPI inhibitor. In this case, the factor VIIa and the factor XIII should be administered sequentially, preferably within about 1-2 hours of each other, for example within 30 minutes of each other or, preferably, within 10 minutes or, more preferred, within 5 minutes of each other. Either of the two unit dosage forms can be administered first.

Since the present invention relates to the prevention or treatment of bleeding episodes or for coagulative treatment by treatment with a combination of active ingredients that may be administered separately, the combination of separate pharmaceutical compositions in kit form is also described. The kit includes at least two separate pharmaceutical compositions. The kit includes container means for containing the separate compositions such as a divided bottle or a divided foil packet. Typically the kit includes directions for the administration of the separate components. The kit form is particularly advantageous when the separate components are preferably administered in different dosage forms, are administered at different dosage intervals, or when titration of the individual components of the combination is desired by the prescribing physician.

Assays

Test for factor VIIa activity:

A suitable assay for testing for factor VIIa activity and thereby selecting suitable factor VIIa variants can be performed as a simple preliminary *in vitro* test:

*In Vitro* Hydrolysis Assay

Native (wild-type) factor VIIa and factor VIIa variant (both hereafter referred to as "factor VIIa") may be assayed for specific activities. They may also be assayed in parallel to directly compare their specific activities. The assay is carried out in a microtiter plate (MaxiSorp, Nunc, Denmark). The chromogenic substrate D-Ile-Pro-Arg-*p*-nitroanilide (S-2288, Chromogenix, Sweden), final concentration 1 mM, is added to factor VIIa (final concentration 100 nM) in 50 mM Hepes, pH 7.4, containing 0.1 M NaCl, 5 mM $CaCl_2$ and 1 mg/ml bovine serum albumin. The absorbance at 405 nm is measured continuously in a SpectraMax™ 340 plate reader (Molecular Devices, USA). The absorbance developed during a 20-minute incubation, after subtraction of the absorbance in a blank well containing no enzyme, is used to calculate the ratio between the activities of variant and wild-type factor VIIa:

$$\text{Ratio} = (A_{405\ nm}\ \text{factor VIIa variant})/(A_{405\ nm}\ \text{factor VIIa wild-type}).$$

Based thereon, factor VIIa variants with an activity comparable to or higher than native factor VIIa may be identified, such as, for example, variants where the ratio between the activity of the variant and the activity of native factor VII shown in Fig. 1 is around, versus above 1.0.

The activity of factor VIIa or factor VIIa variants may also be measured using a physiological substrate such as factor X, suitably at a concentration of 100-1000 nM, where the factor Xa generated is measured after the addition of a suitable chromogenic substrate (eg. S-2765). In addition, the activity assay may be run at physiological temperature.

The ability of factor VIIa or factor VIIa variants to generate thrombin can also be measured in an assay comprising all relevant coagulation factors and inhibitors at physiological concentrations (minus factor VIII when mimicking hemophilia A conditions) and activated platelets (as described on p. 543 in Monroe et al. (1997) Brit. J. Haematol. 99, 542-547 which is hereby incorporated as reference).

Test for factor XIII activity:

A suitable assay for testing for factor XIII transglutaminase activity and thereby selecting suitable factor XIII variants can be performed as a simple *in vitro* test as described, for example, in Methods of Enzymology, Vol. 45 (1976), Proteolytic Enzymes, Part B, pages 177-191 (Ed. Lorand, L.).

The present invention is further illustrated by the following examples, which, however, are not to be construed as limiting the scope of protection. The features disclosed in the foregoing description and in the following examples may, both separately and in any combination thereof, be material for realizing the invention in diverse forms thereof.

EXAMPLES

Example 1.

factor XIII enhances factor VIIa-induced fibrin clot formation.

Citrated normal human plasma (NHP) was diluted 1/10 in buffer containing 20 nM HEPES, 150 mM NaCl, 5 mM $CaCl_2$, pH 7.4 in a micro titer well (total volume 250 µl) and fibrin clot formation was monitored by the increase in optical density at 600 nm in a Specramax™ 340 (Molecular Devices, Sunnyvale CA).

Spontaneous clot formation was obtained at about 2500-3000 sec. Fig. 1 shows that 10 nM recombinant factor VIIa (rFVIIa) (Novo Nordisk A/S Bagsvaerd, Denmark) shortened the clotting time to 1600 sec (n = 2). Further shortening of the clotting time was obtained when 30 nM factor XIII (FXIII) (American Diagnostica inc, Greenwich, CT) was added together with 10 nM rFVIIa (n = 3). The clot formed in the presence of FXIII was more transparent (lower maximal OD) than in its absence indicating that the addition of FXIII resulted in a more fine-meshed fibrin gel structure with thinner fibres.

Example 2.

The presence of supplementary factor XIII during factor VIIa-induced clot formation results in increased resistance to fibrinolytic degradation.

A fibrin clot consisting of thin fibres is mechanically stronger and more difficult to degrade than a clot containing the same amount of fibrin arranged as thick fibres or less cross-linked fibres. The experiment shown in Fig. 2 illustrates that supplementary FXIII (30 nM) prolongs the fibrin clot lysis time of clots formed in the presence of rFVIIa and tissue plasminogen activator (t-PA, American Diagnostica). Clot formation was induced in the presence or absence of 30 nM FXIII by addition of 25 µl NHP to 225 µl 20 nM HEPES, 150 mM NaCl, 5 mM $CaCl_2$, pH 7.4 containing 50 nM rFVIIa and 0.5 nM recombinant t-PA. Clot formation and subsequent clot lysis induced by t-PA-mediated plasminogen activation was monitored by a Spectramax® 340 at 600 nm as the increase in $OD_{600\,nm}$ followed by reversion of the trace to the basal level. Fig. 2 shows that the clot lysis time under these conditions was significantly prolonged by the presence of FXIII.

Example 3.

factor VIIa in combination with factor XIII increases maximal clot firmness and increases clot resistance to fibrinolysis.

Thrombelastograph measurements was conducted on citrated normal human plasma added 6 nM recombinant tissue-type plasminogen activator (t-PA, American Diagnostica) and the effect of addition of 1 nM rFVIIa (Novo Nordisk A/S, Bagsvaerd, Denmark) alone or in combination with various concentrations of factor XIII (FXIII, Haematologic Technologies, HCXIII-0160, Lot N1212,) was analyzed. Clotting was initiated by addition of Innovin (final concentration 2000-fold diluted, Dade Behring # 526945) and calcium (final concentration 15 mM) in a 20 mM HEPES, 150 mM NaCl, pH 7.4 buffer.
Thrombelastograph measurements were utilized to analyze the effect of rFVIIa and FXIII on Maximal Clot Firmness (MCF) as well as clot resistance to t-PA mediated lysis. Prior to rFVIIa and/or FXIII addition the MCF obtained was 25 mm and the time required for half the clot to be lysed was 12.3 minutes (Fig. 3). Addition of increasing concentrations of FXIII (0 - 40 nM) did not alter MCF; however, a dose-dependent prolongation of the half-clot lysis was observed, optimal at 30 nM FXIII (half-clot lysis time: 14,3 min, Fig. 3). Similarly, rFVIIa (1 nM) addition resulted in clot protection from t-PA-mediated fibrinolysis (half-clot lysis time; 16.4 min) without any effect on MCF (Fig. 3). However, upon addition of rFVIIa (1 nM) together with FXIII (30 nM) an increase in the MCF (29 mm), as well as a profound protection from fibrinolysis (half-clot lysis time; 27.1 min) was observed (Fig. 3). Taken together, these results demonstrate that rFVIIa and FXIII addition to plasma in a synergistic fashion improve clot mechanical strength and resistance to t-PA mediated fibrinolysis.

**Claims**

1. Use of a factor VIIa in combination with a factor XIII for the manufacture of a medicament for treating bleeding episodes in a subject, the medicament being for intravenous administration.

2. Use according to claim 1, for the manufacture of a medicament for reducing clotting time in a subject.

3. Use according to claim 1, for the manufacture of a medicament for prolonging the clot lysis time in normal mammalian plasma.

4. Use according to claim 1, for the manufacture of a medicament for increasing clot strength in normal mammalian plasma.

5. Use according to claim 1, for the manufacture of a medicament for enhancing fibrin clot formation in normal human plasma.

6. Use according to any one of claims 1 to 5 , wherein the factor VIIa is a factor VII variant.

7. Use according to claim 6, wherein the factor VII variant is selected from the list of: [L305V]-FVIIa, [L305V/M306D/D309S]-FVIIa, [L305I]-FVIIa, [L305T]-FVIIa, [F374P]-FVIIa, [V158T/M298Q]-FVIIa, [V158D/E296V/M298Q]-FVIIa and [K337A]-FVIIa.

8. Use according to any one of claims 1 to 5, wherein the factor VIIa is human factor VIIa, such as recombinant human factor VIIa.

9. Use according to claim 8, wherein the factor VIIa and the factor XIII is recombinant human factor VIIa and recombinant human factor XIII.

10. Use according to any one of claims 1 to 9, wherein the medicament further contains an inhibitor of the fibrinolytic system.

11. Use according to claim 10, wherein the inhibitor of the fibrinolytic system is selected from the list of: aprotinin, epsilon-aminocaproic acid, and tranexamic acid.

12. Use according to any one of claims 1 to 11, wherein the factor VIIa and the factor XIII is to be administered sequentially within 1-2 hours, such as within 30 minutes, or 10 minutes, of each other.

13. Use according to any one of claims 1 to 12, wherein the subject has an impaired thrombin generation.

14. Use according to any one of claims 1 to 13, wherein the subject has a lowered plasma concentration of fibrinogen.

15. Use according to claim 14, wherein the subject is multi-tranfused, e.g., as a result of multiple trauma or extensive surgery.

16. Use according to claim 15, wherein the multi-tranfusion is a consequence of multiple trauma or extensive surgery.

**Patentansprüche**

1. Verwendung eines Faktors VIIa in Kombination mit einem Faktor XIII zur Herstellung eines Medikaments zur Behandlung von Blutungsvorfällen bei einem Patienten, wobei das Medikament zur intravenösen Verabreichung bestimmt ist.

2. Verwendung nach Anspruch 1 zur Herstellung eines Medikaments zur Reduzierung der Gerinnungszeit bei einem Patienten.

3. Verwendung nach Anspruch 1 zur Herstellung eines Medikaments zum Verlängern der Auflösungszeit eines Gerinnsels in normalem Säugerplasma.

4. Verwendung nach Anspruch 1 zur Herstellung eines Medikaments zur Erhöhung der Gerinnselstärke in normalem Säugerplasma.

5. Verwendung nach Anspruch 1 zur Herstellung eines Medikaments zur Verbesserung der Fibrin-Cerinnselbildung in normalem Säugerplasma.

6. Verwendung nach einem der Ansprüche 1 bis 5, wobei der Faktor VIIa eine Variante des Faktors VII ist.

7. Verwendung nach Anspruch 6, wobei die Variante des Faktors VII ausgewählt ist aus der Liste: [L305V]-FVIIa, [L305V/M306D/D309S]-FVIIa, [L305I]-FVIIa, [L305T]-FVIIa, [F364P]-FVIIa, [V158T/M298Q]-FVIIa, [V158D/E296V/M298Q]-FVIIa und [K337A]-FVIIa.

8. Verwendung nach einem der Ansprüche 1 bis 5, wobei der Faktor VIIa menschlicher Faktor VIIa wie relcombinanter menschlicher Faktor VIIa ist.

9. Verwendung nach Anspruch 8, wobei der Faktor VIIa und der Faktor XIII rekombinanter menschlicher Faktor VIIa und rekombinanter menschlicher Faktor XIII ist.

10. Verwendung nach einem der Ansprüche 1 bis 9, wobei das Medikament ferner einen Hemmstoff des fibrinolytischen Systems enthält.

**11.** Verwendung nach Anspruch 10, wobei der Hemmstoff des fibrinolytischen Systems ausgewählt ist aus der Liste: Aprotinin, epsilon-Aminocapronsäure und Tranexamsäure.

**12.** Verwendung nach einem der Ansprüche 1 bis 11, wobei der Faktor VIIa und der Faktor XIII aufeinander folgend innerhalb von 1-2 Stunden wie innerhalb von 30 Minuten oder 10 Minuten zu verabreichen ist.

**13.** Verwendung nach einem der Ansprüche 1 bis 12, wobei der Patient eine beeinträchtigte Thrombinbildung aufweist.

**14.** Verwendung nach einem der Ansprüche 1 bis 13, wobei der Patient eine verminderte Plasmakonzentration an Fibrinogen aufweist.

**15.** Verwendung nach Anspruch 14, wobei der Patient z.B. als Ergebnis von mehrfachem Trauma oder umfassender Operation mehrfach transfundiert ist.

**16.** Verwendung nach Anspruch 15, wobei die mehrfache Transfusion eine Folge von mehrfachem Trauma oder umfassender Operation ist.


**Revendications**

**1.** Utilisation d'un facteur VIIa en combinaison avec un facteur XIII pour la fabrication d'un médicament destiné à traiter des épisodes de saignement chez un sujet, le médicament étant destiné à une administration intraveineuse.

**2.** Utilisation selon la revendication 1, pour la fabrication d'un médicament destiné à réduire le temps de coagulation chez un sujet.

**3.** Utilisation selon la revendication 1, pour la fabrication d'un médicament destiné à prolonger le temps de lyse de caillot dans un plasma de mammifère normal.

**4.** Utilisation selon la revendication 1, pour la fabrication d'un médicament destiné à augmenter la force de caillot dans un plasma de mammifère normal.

**5.** Utilisation selon la revendication 1, pour la fabrication d'un médicament destiné à accentuer la formation de caillot de fibrine dans un plasma humain normal.

**6.** Utilisation selon l'une quelconque des revendications 1 à 5, dans laquelle le facteur VIIa est un variant du facteur VII.

**7.** Utilisation selon la revendication 6, dans laquelle le variant du facteur VII est sélectionné parmi la liste suivante : [L305V]-FVIIa, [L305V/M306D/D309S]-FVIIa, [L305I]-FVIIa, [L305T]-FVIIa, [F374P]-FVIIa, [V158T/M298Q]-FVIIa, [V158D/E296V/M298Q]-FVIIa et [K337A]-FVIIa.

**8.** Utilisation selon l'une quelconque des revendications 1 à 5, dans laquelle le facteur VIIa est un facteur VIIa humain, tel que le facteur VIIa humain recombinant.

**9.** Utilisation selon la revendication 8, dans laquelle le facteur VIIa et le facteur XIII sont un facteur VIIa humain recombinant et un facteur XIII humain recombinant.

**10.** Utilisation selon l'une quelconque des revendications 1 à 9, dans laquelle le médicament contient de plus un inhibiteur du système fibrinolytique.

**11.** Utilisation selon la revendication 10, dans laquelle l'inhibiteur du système fibrinolytique est sélectionné parmi la liste suivante : aprotinine, acide epsilon-aminocaproïque, et acide tranexamique.

**12.** Utilisation selon l'une quelconque des revendications 1 à 11, dans laquelle le facteur VIIa et le facteur XIII sont à administrer séquentiellement en 1 à 2 heures, comme en 30 minutes, ou 10 minutes, l'un après l'autre.

**13.** Utilisation selon l'une quelconque des revendications 1 à 12, dans laquelle le sujet présente une génération de thrombine affectée.

**14.** Utilisation selon l'une quelconque des revendications 1 à 13, dans laquelle le sujet a une concentration en fibrinogène dans le plasma diminuée.

**15.** Utilisation selon la revendication 14, dans laquelle le sujet est multi-transfusé, par exemple en résultat de multiples traumas ou d'une chirurgie extensive.

**16.** Utilisation selon la revendication 15, dans laquelle la multi-transfusion est une conséquence de multiples traumas ou d'une chirurgie extensive.

Figure 1

OD 600 nm

Figure 2

EP 1 282 438 B1

18

**Figure 3.**